# EUROPEAN PATENT SPECIFICATION

(11) **EP 2 012 754 B1**
(45) Date of publication and mention of the grant of the patent: **05.06.2013**
(21) Application number: 07728399.2
(22) Date of filing: 23.04.2007
(51) Int. Cl.: A61K 9/16, A61K 9/20, A61K 9/48, A61K 31/4439, A61P 1/04

(54) **ORAL RAPID RELEASE PHARMACEUTICAL FORMULATION FOR ESOMEPRAZOLE**
ORALE PHARMAZEUTISCHE FORMULIERUNG MIT SCHNELLER FREISETZUNG FÜR ESOMEPRAZOL
FORMULATION PHARMACEUTIQUE À LIBÉRATION RAPIDE ORALE POUR ESOMEPRAZOLE

(30) Priority: 24.04.2006 CH 6732006
(43) Date of publication of application: 14.01.2009
(73) Proprietor: Mepha Schweiz AG, 4147 Aesch (CH)
(72) Inventor: REHER, Markus, 4102 Binningen (CH); LÜTOLF, Walter, CH-4312 Magden (CH); SPITZ, Marco, 79589 Binzen (DE)
(74) Representative: Braun, André jr.
(86) International application number: PCT/EP2007/053942
(87) International publication number: WO 2007/122212

(56) References cited:
- WO-A-02/45686
- WO-A-94/25070
- WO-A-03/009846
- WO-A-03/072048
- WO-A-2005/004921

## Description

The invention concerns a pharmaceutical formulation in solid, oral, rapid release, dosage form comprising 10 % to 40 % by weight of esomeprazole and 5 % to 50 % by weight of carrageenan.

In patent publication EP 5 129 substituted 2-(pyridylmethylsulfinyl)-1H-benzimidazoles, so-called prazoles, e.g. the nowadays commercially obtainable omeprazole, pantoprazole, lansoprazole und rabeprazole, and their properties as efficient proton pump inhibitors were described. Esomeprazole is the designation for the S enantiomer of omeprazole, S-5-methoxy-2-[[(4-methoxy-3,5-dimethyl-2-pyridinyl)methyl]sulfinyl]-1H-benzimidazole. Pyridylmethylsulfinyl-benzimidazoles are active as secretion inhibitors of gastric acid and are useful as anti-ulcus medicaments and for the prevention and treatment of diseases connected with excess production of gastric acid in mammals and, in particular, in humans.

In patent application WO03/009846 a chewable tablet comprising 10 or 20 mg of omeprazole and carrageenan was disclosed. However, no amounts/percentages of carrageenan were mentioned in this document.

It is known that pyridylmethylsulfinyl-benzimidazoles are comparatively unstable in the presence of humidity, but also in the presence of organic solvents. They are particularly unstable under acid conditions. A possible option for the preparation of derivatives with increased stability is the formation of alkaline salts, e.g. sodium or magnesium salts, for example as described for omeprazole in patent publication EP 124 495.

For the oral application of esomeprazole a pharmaceutical dosage form is preferably so chosen as to ensure that the active ingredient can pass the acid environment of the stomach without damage. Such dosage forms are known and are, for example, pharmaceutical formulations with slow release of the active ingredient also in acid environment, or formulations coated with an acid stable layer dissolving under neutral or slightly alkaline conditions and releasing the active ingredient in the gut.

According to WO 94/27988 esomeprazole is generally formed as an amorphous solid in the form of a syrup or a viscous oil during synthesis or on release from its alkaline salts. Alkaline salts of racemic omeprazole and of the S enantiomer, for example sodium and magnesium salts, are present in crystalline form and have a higher stability for this reason. Particularly suitable is esomeprazole magnesium salt dihydrate or esomeprazole magnesium salt trihydrate. As described in WO 98/028294 it is also possible to prepare stable crystalline forms of esomeprazole itself.

Possibilities have been evaluated to make available stable oral pharmaceutically acceptable dosage forms rapidly releasing the active ingredient from crystalline or amorphous esomeprazole or alkaline salts of esomeprazole, with sufficient dosage precision, under economical conditions, and with optimised chemical and physical stability of the active ingredient and additives.

It has now surprisingly been found that solid oral dosage forms comprising esomeprazole, carrageenan and, if desired, an excipient, for example mannitol and/or microcrystalline cellulose, release the active ingredient unexpectedly rapid.

The invention relates to pharmaceutical compositions in solid oral dosage form, for example pellets, tablets, mini-tablets or capsules, comprising esomeprazole and carrageenan, and further to such solid oral dosage forms that contain additional excipients. Furthermore, the invention comprises the named solid oral dosage forms, for example pellets or mini-tablets, coated with a polymer dissolving only at a pH value of 5 or higher, optionally over a stabilising intermediate coating-layer. The invention likewise comprises pellets or mini-tablets pressed to tablets wherein the tablets are coated with a polymer dissolving only at pH 5 or higher. The invention further comprises capsules, which contain such coated pellets or mini-tablets that dissolve only at pH 5 or higher, or capsules that are coated themselves with a polymer dissolving only at pH 5 or higher. Furthermore, the invention relates to the named oral pharmaceutical compositions for use as a medicament as defined in claim 14.

Particularly useful are the dosage forms of the invention for esomeprazole in the form of an alkaline salt, for example magnesium or sodium salt. Preferably esomeprazole in crystalline or amorphous form is used, or as a magnesium salt dihydrate or trihydrate. Highly preferably, esomeprazole magnesium salt dihydrate is used.

Carrageenan is a polysaccharide with high absorbing capacity for water, manufactured from red algae. κ-Carrageenan consists of repetitive basic units of a disaccharose formed from galactose-4-sulfate and anhydrogalactose. Several variants of carrageenan are available on the market, and are, for example, offered by the firms Degussa, Germany, FMC Biopolymers, Pennsylvania, USA, and CPCelco, Denmark, under several different trade names. Carrageenan is sometimes used in pharmaceutical compositions due to its physical properties as a binder and thickening agent. In the composition according to the invention carrageenan is not only active as a binder allowing or facilitating the manufacture of pellets, but surprisingly also active as a disintegrant allowing rapid and even release of the active ingredient.

Excipients are, for example, solid powdery carriers with low water content. Examples of suitable excipients are sugar alcohols, for example mannitol, xylitol or Sorbitol, sugars, for example mannose, lactose, fructose, glucose, sucrose, or saccharose, cellulose and cellulose derivatives, for example microcrystalline cellulose, hydroxypropylcellulose (hyprolose), hydroxypropylmethylcellulose (hypromellose), mixtures and compounds, respectively, from cellulose derivatives with other excipients (e.g. silicium dioxide, xanthan, guar gum, carboxymethylcellulose sodium), natural or modified starches, for example corn starch or potato starch, silicium dioxide and silicates, for example magnesium aluminium silicate, calcium silicate, and phosphates, such as calcium and magnesium phosphate.

Suitable excipients for the stabilisation of acid labile esomeprazole are, in particular, buffers with a pH value of 6 or higher, preferably between pH 8 and 11, for example buffer mixtures comprising sodium phosphate; sodium hydrogen phosphate, sodium dihydrogen phosphate, sodium carbonate, sodium hydrogen carbonate, sodium hydroxide, or combinations of the named compounds.

Further stabilising excipients are alkaline organic compounds containing nitrogen, for example alkaline amino acids, such as the natural alkaline amino acids lysine or arginine, simple organic bases such as ethylenediamine, ethanolamine, propanolamine, ethyleneamine or ethanolamine further substituted at the nitrogen atom, e.g. N,N'-dibenzyl-ethyleneamine or choline, amino sugars, e.g. meglumine, procaine (4-aminobenzoic acid diethylaminoethyl ester), chloroprocaine or procainamide, and mixtures of the named compounds.

Further excipients considered are diluents, e.g. calcium carbonate, calcium sulfate, hydrogenated vegetable oil, kaolin, magnesium carbonate, talcum, calcium hydrogen phosphate, or sodium chloride, other organic excipients that are suitable as binders and thickening agents in addition to carrageenan, e.g. guar gum, gelatine, polyvinylpyrrolidone, hydroxypropylmethylcellulose, hydroxyethylcellulose, hydroxypropylcellulose, traganth, alginate, carboxymethylcellulose calcium or sodium, or xanthane, disintegrants, which support or enforce the corresponding property of carrageenan, e.g. croscarmellose, crospovidone (crosslinked polyvinylpyrrolidone), colloidal silicium dioxide, sodium starch glycolate, or sodium carboxymethyl starch, glidants, e.g. colloidal silicium dioxide, starch, tribasic calcium phosphate, or talcum, and lubricants, e.g. calcium stearate, zinc stearate, magnesium stearate, stearic acid, fumaric acid, glycerol monostearate, glycerol palmitostearate, mineral oil, sodium benzoate, sodium laurylsulfate, sodium stearylfumarate, talcum, solidified ricinus oil, or hydrogenated castor oil.

In contrast to the formulation described in WO 1996/37195 no titanium dioxide is used according to the present invention.

Pharmaceutical dosage forms according to the invention are, for example, pellets, i.e. globules, flattened globules, or corresponding oval and elliptic forms with a diameter of from 0.3 to 2.0 mm, preferably 0.5 to 1.0 mm, ideally spherical globules. The globules have an aspect ratio of 1 to 2, preferably 1.0 to 1.5, particularly preferred of 1.0 to 1.2. Such pellets are obtained by mixing the starting materials followed by addition of suitable solvents, for example water, ethanol, isopropanol, or propylene glycol. The moist mass obtained thereby is extruded with a suitable apparatus, the extrudates spheronized with a suitable apparatus (a spheronizer), and then dried.

A precondition for the formation of pellets is a suitable composition of the excipients that form a mass together with the active ingredient, which shows the required stickiness, adhesivity, elasticity, and plasticity to combine into solid forms under slight pressure, which forms demonstrate the required physical stability in order to be further processed, for example filled into capsules, coated, and/or pressed into tablets.

Further pharmaceutical dosage forms according to the invention are mini-tablets, i.e. spherical, elliptic or oval discs with curved surfaces or cylindrical bodies with a diameter of between 0.5 and 4.0 mm, preferably 1.0 and 3.0 mm. Such mini-tablets are obtained e.g. by direct pressing of a mixture of the active ingredient and carrageenan with optionally further suitable excipients in a tablet press. In principle, there is the option that the mixture is compacted before being pressed into mini-tablets, or also granulated, i.e. a solvent is added that is removed later by drying.

Excipients are chosen in a way that the mini-tablets will show the required physical stability after pressing, in order to be, for example, coated and filled into capsules.

Particularly suitable pharmaceutical compositions in form of pellets are those comprising, besides the active ingredient and carrageenan, a sugar alcohol, for example mannitol, or microcrystalline cellulose as excipients. Particularly suitable pharmaceutical compositions in form of mini-tablets are those comprising, besides the active ingredient and carrageenan, dicalcium phosphate and magnesium stearate as excipients. The compositions comprise 10-40% active ingredient and 5-50% carrageenan, in particular 25-35% active ingredient and 9-20% carrageenan, whereby the preferred active ingredient is esomeprazole as an amorphous compound or as the crystalline alkaline magnesium salt dihydrate. Particularly preferred are pellets with a composition of approx. 30% esomeprazole, approx. 10% carrageenan, mannitol, and/or microcrystalline cellulose, and optionally further excipients, for example buffer compounds. Likewise particularly preferred are mini-tablets with a composition of approx. 30% esomeprazole, approx. 10% carrageenan, microcrystalline cellulose, magnesium stearate, and optionally further excipients.

The pellets or mini-tablets according to the invention may be coated or directly filled into capsules. Capsules considered are soft gelatine, hard gelatine, HPMC, polysaccharide or starch capsules as plugged, welded or glued capsules, of different size, colour, and water content.

if desired the pellets, mini-tablets, tablets, or capsules of the invention are coated with a polymer dissolving only at a pH value of 5 or higher, if desired after coating with a stabilising intermediate layer.

Such polymer coatings, which dissolve only at a pH value of 5 or higher, are, for example, ethylcellulose, cellulose acetate phthalate, shellac, hydroxypropylmethylcellulose acetate succinate, and methacrylic acid polymers.

It is also possible to apply an intermediate layer before coating with the coating material stable up to pH 5, which inhibits or diminishes the interaction of the active ingredient and/or added buffer compounds or alkaline organic compounds with the coating. Suitable stabilising intermediate layers are, for example, hydroxypropylmethylcellulose, hydroxypropylcellulose, polyvinylpyrrolidone, or polyvinyl alcohol.

It has now surprisingly been found that the pharmaceutical compositions according to the invention do not only show a sufficient physical and chemical stability, but also rapidly release the active ingredient.

The following examples illustrate the invention, but do not represent a limitation of the subject matter of the invention.

### Example 1:

| Substance | | Amount per unit | Amount per batch of 10000 units |
|---|---|---|---|
| Esomeprazole magnesium dihydrate | 30% | 44.25 mg | 442.5 g |
| Mannitol | 40% | 59.00 mg | 590.0 g |
| Microcrystalline cellulose | 20% | 29.50 mg | 295.0 g |
| Carrageenan* | 10% | 14.75 mg | 147.5 g |
| Water | | 71.09 mg | 710.9 g |

| | | | |
|---|---|---|---|
| * Gelcarin® GP 812 NF from FMC Biopolymers | | | |

Manufacture: Esomeprazole magnesium dihydrate is mixed with mannitol, microcrystalline cellulose, and carrageenan (type Gelcarin® GP 812 NF from FMC) in the amounts indicated above for 10 minutes in a mixer of the firm Colette. Water is added thereafter with stirring at a constant rate during a period of further 10 minutes. The moist mass is pressed through a perforated disc with boreholes of 1.0 mm diameter in an extruder of the firm Probst. The so-called extrudate is spheronized thereafter in a spheronizer of the firm Caleva during 4 minutes with a disc rotation velocity of 1050 rpm. The obtained pellets are dried in a fluidised bed dryer of the firm Aeromatik during 160 minutes and a supply air temperature of 65°C.

### Comparative example A:

| | |
|---|---|
| Esomeprazole magnesium dihydrate | 30% |
| Mannitol | 40% |
| Microcrystalline cellulose | 20% |
| Crospovidone | 8% |
| Hypromellose 3 mPas | 2% |

Pellets are manufactured in analogy to example 1. In place of carrageenan a disintegrant (crospovidone) and a binder (hypromellose) are added.

### Comparative example B:

| | |
|---|---|
| Esomeprazole magnesium dihydrate | 30% |
| Mannitol | 40% |
| Microcrystalline cellulose | 20% |
| sodium carboxymethyl starch | 8% |
| Hypromellose 3 mPas | 2% |

Pellets are manufactured in analogy to example 1. In place of carrageenan a disintegrant (sodium carboxymethyl starch) and a binder (hypromellose) are added.

### Measurement of the release of the active ingredient

Pellets manufactured according to example 1 and comparative examples A and B, respectively, are stirred at pH 8.6 and 100 rpm at 37 ± 0.5 °C according to the paddle method, and the amount of dissolved active ingredient determined after pre-determined time periods by measuring the spectrophotometric absorption at 300 nm (Perkin Elmer Lambda 15) and comparing with a reference solution.

**Table 1: Comparison of carrageenan with other disintegrants in pellets**

| time | Example 1 | Comparative example A | Comparative example B |
|---|---|---|---|
| | **Carrageenan** | Crospovidone | Sodium carboxymethyl starch |
| 10 min | **52.9%** | 27.9% | 32.3% |
| 20 min | **77.8%** | 41.9% | 49.8% |
| 30 min | **90.8%** | 51.7% | 61.9% |

The results of the measurement demonstrate that the active ingredient is released much more rapidly from pellets of example 1 than from pellets composed according to comparative examples A and B.

### Example 2:

| | |
|---|---|
| Esomeprazole magnesium dihydrate | 28.2% |
| Sorbitol | 37.4% |
| Hydroxypropylcellulose | 18.7% |
| Carrageenan | 9.3% |
| Sodium hydrogen carbonate | 4.5% |
| Sodium hydroxide | 1.9% |

Pellets are manufactured in analogy to example 1. In addition the formulation comprises a mixture of sodium hydrogen carbonate and sodium hydroxide as a buffer. The buffer is dissolved in water.

### Example 3:

| | |
|---|---|
| Esomeprazole magnesium dihydrate | 28.2% |
| Mannitol | 27.5% |
| Microcrystalline cellulose | 18.7% |
| Carrageenan | 19.2% |
| Sodium hydrogen carbonate | 4.5% |
| Sodium hydroxide | 1.9% |

Pellets are manufactured in analogy to example 1. In comparison to example 2 the amount of carrageenan was doubled at the expense of mannitol.

### Examples 4 to 8

Manufacturing example 4: Esomeprazol magnesium dihydrate is mixed with dicalcium phosphate, carrageenan and magnesium stearate in the amounts indicated below for 10 minutes in a mixer of the firm Colette. The mixture is pressed into mini-tablets in a concentric tabletting apparatus of the firm Korsch with a disc diameter of 2.5 mm. Mini-tablets of examples 5 to 8 are manufactured analogously.

Amounts in mg per unit (in working examples converted to 10000 units)

| | Example 4 | Example 5 | Example 6 | Example 7 | Example 8 |
|---|---|---|---|---|---|
| Esomeprazole magnesium dihydrate | 44.25 mg | 44.25 mg | 44.25 mg | 43.38 mg | 43.38 mg |
| Dicalcium phosphate | 50.00 mg | 35.00 mg | 50.00 mg | 28.62 mg | - |
| Trisodium citrate | - | - | 26.88 mg | - | - |
| Carrageenan | 53.75 mg **35.8%** | 68.75 mg **45.8%** | 26.88 mg **17.9%** | 15.00 mg **10.7%** | 15.00 mg **10.7%** |
| Alkaline granulated microcrystalline cellulose | -- | -- | -- | 50.00 mg | 78.62 mg |
| Magnesium stearate | 2.00 mg | 2.00 mg | 2.00 mg | 3.00 mg | 3.00 mg |
| Total | 150.00 mg | 150.00 mg | 150.00 mg | 140.00 mg | 140.00 mg |

### Comparative examples C to E

Amounts in mg per unit (in working examples converted to 10000 units)

| | Example C | Example D | Example E |
|---|---|---|---|
| Esomeprazole magnesium dihydrate | 44.25 mg | 44.25 mg | 44.25 mg |
| Dicalcium phosphate | 119.48 mg | 88.48 mg | 55.30 mg |
| Calcium silicate | -- | 30.97 mg | 64.15 mg |
| Magnesium stearate | 2.21 mg | 2.21 mg | 2.21 mg |
| Total | 165.90 mg | 165.90 mg | 165.90 mg |

Manufacturing comparative example C: Esomeprazol magnesium dihydrate is mixed with dicalcium phosphate and magnesium stearate in the amounts indicated for 10 minutes in a mixer of the firm Colette. The mixture is pressed into mini-tablets in a concentric tabletting apparatus of the firm Korsch with a disc diameter of 2.5 mm. Mini-tablets of comparative examples D and E are manufactured analogously.

### Measurement of the release of the active ingredient

Mini-tablets manufactured according to examples 4 to 8 and comparative examples C to E, respectively, are stirred at pH 8.6 and 100 rpm at 37 ± 0.5°C according to the paddle method, and the amount of dissolved active ingredient determined after pre-determined time periods by measuring the spectrophotometric absorption at 300 nm (Perkin Elmer Lambda 15) and comparing with a reference solution.

**Table 2: Dissolution of mini-tablets of examples 4 to 8**

| Time | Example 4 | Example 5 | Example 6 | Example 7 | Example 8 |
|---|---|---|---|---|---|
| 5 min | 94.5% | 94.2% | 96.4% | 103.4% | 88.9% |
| 10 min | 98.8% | 99.1% | 100.0% | 105.4% | 96.1% |
| 20 min | 99.9% | 100.2% | 100.8% | 105.1% | 96.1% |
| 30 min | 100.4% | 100.6% | 101.2% | 105.0% | 96.0% |

The results of the measurement demonstrate that the active ingredient is released extremly rapid from mini-tablets with carrageenan. The amount of carrageenan has no substantial effect on the dissolution in the examined range of 10-45%. In all cases the release is more than 95% after 10 minutes.

**Table 3: Dissolution of mini-tablets of comparative examples C to E without carrageenan**

| time | Comparative example C | Comparative example D | Comparative example E |
|---|---|---|---|
| 5 min | 15.6% | 16.0% | 18.0% |
| 10 min | 29.6% | 32.1% | 35.6% |
| 15 min | 43.3% | 46.7% | 51.1% |
| 20 min | 53.0% | 57.3% | 61.9% |
| 30 min | 65.6% | 72.1% | 76.1% |
| 40 min | 71.1% | 79.4% | 81.6% |
| 50 min | 77.8% | 87.2% | 87.1% |
| 60 min | 78.7% | 88.5% | 87.6% |

The results of the measurement demonstrate that the active ingredient is released comparatively slow from mini-tablets without carrageenan.

## Claims

1. A pharmaceutical composition in solid, oral, rapid release, dosage form comprising 10 % to 40 % by weight of esomeprazole and 5 % to 50 % by weight of carrageenan.

2. A pharmaceutical composition according to claim 1, **characterized in that** said esomeprazole is esomeprazole in amorphous form.

3. A pharmaceutical composition according to claim 1, **characterized in that** said esomeprazole is esomeprazole magnesium salt dihydrate.

4. A pharmaceutical composition according to claim 1, **characterized in that** said esomeprazole is esomeprazole magnesium salt trihydrate.

5. A pharmaceutical composition according to any one of claims 1 to 4, **characterized in that** said dosage form is selected from the group consisting of a pellet, a tablet, a mini-tablet and a capsule.

6. A pharmaceutical composition according to claim 5, **characterized in that** said dosage form is a pellet.

7. A pharmaceutical composition according to claim 5, **characterized in that** said dosage form is a mini-tablet.

8. A pharmaceutical composition according to any one of claims 1 to 7 further comprising at least one excipient.

9. A pharmaceutical composition according to any one of claims 1 to 8 further comprising, as an excipient, a buffer mixture with a pH value higher than 6.0.

10. A pharmaceutical composition according to any one of claims 1 to 9 comprising 25 % to 35 % by weight of said esomeprazole and 9 % to 20 % by weight of said carrageenan.

11. A pharmaceutical composition according to any one of claims 1 to 10 coated with a polymer, which dissolves only at a pH value equal to, or higher than, 5.0.

12. A pharmaceutical composition according to any one of claims 1 to 10 in the form of a capsule, or a tablet, coated with a polymer, which dissolves only at a pH value equal to, or higher than, 5.0, comprising at least one pellet, or mini-tablet, as defined in claim 5.

13. A pharmaceutical composition according to claim 11 or claim 12 comprising a stabilizing intermediate layer below the said polymer coating, which dissolves only at a pH value equal to, or higher than, 5.0.

14. A pharmaceutical composition as defined in any one of claims 1 to 13 for use as an anti-ulcus medicament, or for use as a medicament for the prevention, or the treatment, of a disease connected with the excess production of gastric acid, in a mammal, in particular in a human.

## Patentansprüche

1. Pharmazeutische Zusammensetzung in fester, oraler Darreichungsform mit schneller Wirkstofffreisetzung, umfassend 10 Gew.-% bis 40 Gew.-% Esomeprazol und 5 Gew.-% bis 50 Gew.-% Carrageenan.

2. Pharmazeutische Zusammensetzung nach Anspruch 1, **dadurch gekennzeichnet, dass** das Esomeprazol Esomeprazol in amorpher Form ist.

3. Pharmazeutische Zusammensetzung nach Anspruch 1, **dadurch gekennzeichnet, dass** das Esomeprazol Esomeprazolmagnesiumsalzdihydrat ist.

4. Pharmazeutische Zusammensetzung nach Anspruch 1, **dadurch gekennzeichnet, dass** das Esomeprazol Esomeprazolmagnesiumsalztrihydrat ist.

5. Pharmazeutische Zusammensetzung nach einem der Ansprüche 1 bis 4, **dadurch gekennzeichnet, dass** die Darreichungsform aus der Gruppe, bestehend aus einem Pellet, einer Tablette, einer Minitablette und einer Kapsel, ausgewählt ist.

6. Pharmazeutische Zusammensetzung nach Anspruch 5, **dadurch gekennzeichnet, dass** die Darreichungsform ein Pellet ist.

7. Pharmazeutische Zusammensetzung nach Anspruch 5, **dadurch gekennzeichnet, dass** die Darreichungsform eine Minitablette ist.

8. Pharmazeutische Zusammensetzung nach einem der Ansprüche 1 bis 7, ferner umfassend zumindest einen Exzipienten.

9. Pharmazeutische Zusammensetzung nach einem der Ansprüche 1 bis 8, ferner umfassend ein Puffergemisch mit einem pH-Wert von mehr als 6,0 als Exzipient.

10. Pharmazeutische Zusammensetzung nach einem der Ansprüche 1 bis 9, umfassend 25 Gew.-% bis 35 Gew.-% des Esomeprazols und 9 Gew.-% bis 20 Gew.-% des Carrageenans.

11. Pharmazeutische Zusammensetzung nach einem der Ansprüche 1 bis 10, beschichtet mit einem Polymer, das sich erst bei einem pH-Wert von größer gleich 5,0 auflöst.

12. Pharmazeutische Zusammensetzung nach einem der Ansprüche 1 bis 10 in Form einer Kapsel oder einer Tablette, beschichtet mit einem Polymer, das sich erst bei einem pH-Wert von größer gleich 5,0 auflöst, umfassend zumindest ein Pellet oder eine Minitablette nach Anspruch 5.

13. Pharmazeutische Zusammensetzung nach Anspruch 11 oder 12, umfassend eine stabilisierende Zwischenschicht unter der Polymerbeschichtung, die sich erst bei einem pH-Wert von größer gleich 5,0 auflöst.

14. Pharmazeutische Zusammensetzung nach einem der Ansprüche 1 bis 13 zur Verwendung als Anti-Ulcus-Medikament oder zur Verwendung als Medikament für die Prävention oder Behandlung einer Krankheit, die mit der überschüssigen Produktion von Magensäure bei einem Säugetier, insbesondere einem Menschen, in Zusammenhang steht.

## Revendications

1. Composition pharmaceutique sous forme de dosage, à libération rapide, orale, solide, comprenant 10 % à 40 % en poids d'ésoméprazole et 5 % à 50 % en poids de carragénine.

2. Composition pharmaceutique selon la revendication 1, **caractérisée par le fait que** ledit ésoméprazole est un ésoméprazole sous forme amorphe.

3. Composition pharmaceutique selon la revendication 1, **caractérisée par le fait que** ledit ésoméprazole est un sel de magnésium d'ésoméprazole dihydraté.

4. Composition pharmaceutique selon la revendication 1, **caractérisée par le fait que** ledit ésoméprazole est un sel de magnésium d'ésoméprazole trihydraté.

5. Composition pharmaceutique selon l'une quelconque des revendications 1 à 4, **caractérisée par le fait que** ladite forme de dosage est choisie dans le groupe consistant en une pastille, un comprimé, un mini-comprimé et une capsule.

6. Composition pharmaceutique selon la revendication 5, **caractérisée par le fait que** ladite forme de dosage est une pastille.

7. Composition pharmaceutique selon la revendication 5, **caractérisée par le fait que** ladite forme de dosage est un mini-comprimé.

8. Composition pharmaceutique selon l'une quelconque des revendications 1 à 7, comprenant en outre au moins un excipient.

9. Composition pharmaceutique selon l'une quelconque des revendications 1 à 8, comprenant en outre, en tant qu'excipient, un mélange tampon ayant une valeur de pH supérieure à 6,0.

10. Composition pharmaceutique selon l'une quelconque des revendications 1 à 9 comprenant 25 % à 35 % en poids dudit ésoméprazole et 9 % à 20 % en poids de ladite carraghénine.

11. Composition pharmaceutique selon l'une quelconque des revendications 1 à 10 revêtue d'un polymère, qui se dissout seulement à une valeur de pH égale ou supérieure à 5,0.

12. Composition pharmaceutique selon l'une quelconque des revendications 1 à 10 sous la forme d'une capsule enrobée d'un polymère, ou d'un comprimé enrobé d'un polymère, qui se dissout seulement à une valeur de pH égale ou supérieure à 5,0, comprenant au moins une pastille ou un mini-comprimé, comme défini à la revendication 5.

13. Composition pharmaceutique selon la revendication 11 ou la revendication 12 comprenant une couche intermédiaire de stabilisation sous ledit enrobage de polymère, qui se dissout seulement à une valeur de pH égale ou supérieure à 5,0.

14. Composition pharmaceutique selon l'une quelconque des revendications 1 à 13 destinée à être utilisée en tant que médicament anti-ulcéreux ou destinée à être utilisée en tant que médicament pour la prévention ou le traitement d'une maladie liée à la production excessive d'acide gastrique, chez un mammifère, en particulier chez un être humain.
